# EUROPEAN PATENT SPECIFICATION

(11) **EP 3 445 264 B1**
(45) Date of publication and mention of the grant of the patent: **26.05.2021**
(21) Application number: 17726989.1
(22) Date of filing: 18.04.2017
(51) Int. Cl.: A61B 17/80

(54) **IMPLANT FOR MAINTAINING BONE DIASTASIS AFTER OSTEOTOMY AND BONE DEFICIT FILLER STRUCTURE WITH MECHANICAL SUPPORT CAPACITY**
IMPLANTAT ZUR AUFRECHTERHALTUNG VON KNOCHENDIASTASE NACH OSTEOTOMIE UND KNOCHENDEFIZITFÜLLERSTRUKTUR MIT MECHANISCHER STÜTZFÄHIGKEIT
IMPLANT POUR MAINTENIR UNE DIASTASE OSSEUSE APRÈS UNE OSTÉOTOMIE ET STRUCTURE DE REMPLISSAGE DE DÉFICIT OSSEUX AYANT UNE CAPACITÉ DE SUPPORT MÉCANIQUE

(30) Priority: 21.04.2016 IT UA20162794
(43) Date of publication of application: 27.02.2019
(73) Proprietor: Ad Maiora S.r.l., 42025 Cavriago, Reggio Emilia (IT)
(72) Inventor: ROVESTI, Gian Luca, 42021 Barco Di Bibbiano Reggio Emilia (IT); UGOLOTTI, Oscar, 42025 Cavriago Reggio Emilia (IT)
(74) Representative: Casadei, Giovanni
(86) International application number: PCT/IB2017/052196
(87) International publication number: WO 2017/182935

(56) References cited:
- WO-A1-2013/179142
- WO-A2-2013/006778
- US-A- 5 766 251
- US-A1- 2005 075 641

## Description

The present invention relates to an implant for maintaining bone diastasis after tibial crest osteotomy, preferably, but not exclusively, for veterinary use.

Implants of this kind are known for the treatment of the stifle with anterior cruciate ligament (ACL) rupture in the veterinary field.

The ACL is located inside the stifle to stabilise the joint.

In greater detail, the anterior cruciate ligament performs several functions, including those of preventing anterior subluxation of the tibia, limiting excessive extension of the stifle and limiting internal rotation of the tibia on the femur.

Excessive stress during such movements can damage the ligament and cause the progressive degeneration thereof until complete rupture. It follows, therefore, that ACL rupture is a rather frequent event, particularly in dogs.

Many surgical procedures have been proposed for treatment of ACL rupture in the dog. In particular, two fundamental types of surgical intervention are known: intracapsular procedures, which entail opening the stifle joint and stabilising it internally, and extracapsular procedures that stabilise the joint and are performed operating outside the joint space.

The former group of procedures aims replacing the original damaged ligament that is no longer functional with one that may consist of many different materials that replace the previous one in the function of stabilising the stifle.

The latter group of procedures aims modifying the joint load axes in such a way as to create biomechanical conditions that render the function of the ACL during stifle extension unnecessary. Such interventions entail performing osteotomies of the tibia and subsequently stabilising them using various synthetic means.

The tibial crest advancement (TTA) is a surgical procedure that belongs to the second group. It entails the use of a separating body suitable for insertion into the space generated by the tibial crest advancement.

In this procedure, the stress to which the separating body is subjected is very high. For this reason, some procedures provide for a solid separating body, which does not, however, have an osteoconduction capacity. If the separating body is made with a mesh-like or sponge-like structure to enable osteoconduction, the risk is related to the collapse of the implant. On the other hand, increasing the rigidity of the implants renders them less capable of being adapted to individual clinical situations, being not possible to shape or modify them in order to obtain specific adaptations to the patient.

An example of implant is disclosed in document WO2013/179142. Such implant does not solve the drawbacks above summarized.

In this context, the technical task at the basis of the present invention is to propose an implant that enables the aforesaid drawbacks of the prior art to be overcome.

In particular, it is an object of the present invention to provide an implant capable of offering greater resistance to loads and hence to the collapsing of the structure itself, while ensuring optimal osteo-integration.

A further object of the present invention is to propose an implant that enables a particularly stable fastening, for example by means of pins or nails.

A further object of the present invention is to propose an intervention for maintaining bone diastasis after osteotomy, for example of the tibial crest, or ostectomy, and for filling bone deficits, which makes it possible to reduce surgery times and improve flexibility in fastening the implant to the bone structure.

A further object of the present invention is to provide a three-dimensional structure with characteristics such as to permit the insertion of biological materials that favour healing of the adjacent bone tissue. For example, by way of non-exclusive example, the structure can serve as a carrier for cells of osseous origin of varying nature, stem cells, morphogenetic proteins, etc. A further object of the present invention is to provide a structure that can be modified in the course of the operation with the aim of adapting it to specific needs in different situations. For this reason, the three-dimensional meshes can be cut so as to enable a high adaptability in terms of size and shape. The stated technical tasks and specified objects are substantially achieved by an implant comprising the technical features disclosed in one or more of the appended claims. The dependent claims correspond to different possible embodiments of the invention.

In particular, in accordance with a first aspect, the present invention relates to an implant which comprises a separating body made of biocompatible material suitable for insertion into a longitudinal osteotomy. With reference to an engagement configuration of the implant, the separating body extends along a longitudinal direction between an upper portion and a lower portion. The separating body comprises a main section having at least one opening extending along a passage axis disposed according to a direction transverse to the longitudinal direction, with reference to the engagement configuration of the implant. The opening being suitable for favouring osteo-integration and, where it is deemed useful and/or necessary, carrying biological material capable of stimulating the bone response.

The separating body further comprises at least one reinforcement plate disposed at at least one of the upper or side portions transversely to the longitudinal direction.

In accordance with a second aspect, the present invention relates to a tibial crest advancement intervention, for veterinary use, but not only. The intervention comprises the steps of osteotomy of the tibial crest and the subsequent cranial advancement thereof to generate a space between the tibial metaphysis and the crest, in which to introduce the separating body. In order to obtain stabilisation of the tibial crest, it is possible to insert a pin or nail according to an anteroposterior direction, passing through an opening of said separating body and inclining said pin or nail according to the anatomy of the tibia and/or the characteristics of the longitudinal slit.

In accordance with a further aspect, the invention relates to the function of filling and mechanically supporting a bone deficit that may have arisen spontaneously as a result of a disease or can be of iatrogenic origin. In this case the function of the implant is to replace the bone segment that is missing or has been ostectomised for clinical reasons, offering a mechanical support that can establish a relation between the two residual bone stumps. In this context, the technical task at the basis of the present invention is to propose an implant that can maintain the transmission of loads between the residual bone stumps, thus enabling osteo-conduction and subsequent osteo-integration. Of particular importance in this context are the characteristics already described on page 3 of the present document regarding the possibility of the three-dimensional structure of carrying biological material, such as cells or morphogenetic proteins, with a bone stimulation function and the possibility of making specific adaptations of the implant by cutting the meshes of the structure so as to make adaptations of a dimensional and morphological type.

In one or more of the specified aspects, the present invention can comprise one or more of the following features.

Preferably, the separating body is made of titanium.

Preferably, the separating body is produced by electron beam melting. Preferably, the separating body has a wedge-like conformation and, even more preferably, the upper portion has, transversely to the longitudinal direction, a greater extent than the lower portion.

Preferably, at least one reinforcement plate extends transversely to the longitudinal direction on an upper and/or lateral portion of the separating body. According to the invention as claimed, there is provided a plurality of openings disposed parallel to one another. In particular, the openings are disposed along rows. Even more preferably, the openings of two adjacent rows alternate with each other.

In a cross section perpendicular to the passage axis, the openings have an elongate passageway, with its largest dimension disposed along the longitudinal direction.

Preferably, the openings have a hexagonal passageway.

Preferably, there is provided at least one fastening pin or nail suitable for passing through the opening of the separating body.

Additional features and advantages of the present invention will become more apparent from the indicative, and thus non-limiting, description of a preferred but not exclusive embodiment of an implant for maintaining bone diastasis after osteotomy or ostectomy and as a bone deficit filler having a mechanical support capacity and a capacity to carry biostimulating material, for a preferred but non-exclusive veterinary use.

This description will be set out below with reference to the attached drawings, provided solely for illustrative and therefore non-limiting purposes, in which:
- figure 1 schematically illustrates a perspective view of a separating body of an implant according to the present invention;

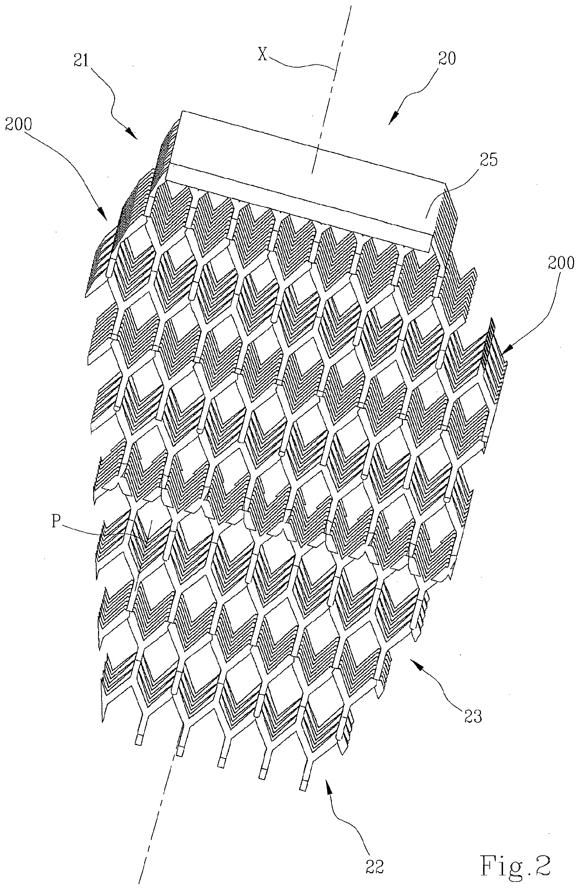

- figure 2 illustrates the separating body of figure 1 according to a different angle;
- figure 3 illustrates an implant according to the present invention in an engagement configuration;
- figure 4 illustrates an implant according to the present invention, in an engagement configuration alternative to the one in figure 3.
- figure 5 illustrates an implant according to the present invention used for maintaining bone diastasis after ostectomy and as a bone deficit filler having a mechanical support capacity and a capacity to carry biostimulating material.

There can be numerous applications of the implant according to the present invention, which possesses the capacity to be inserted in an osteotomy or ostectomy, or in spontaneously occurring situations of bone deficits. The implant according to the present invention can in fact perform a separating function for maintaining the bone diastasis obtained following an osteotomy, or else provide a substitutive function, from a mechanical viewpoint, for the interruption between two bone segments, both when this has occurred spontaneously and when it has been deliberately obtained as part of a more complex procedure. For non-exclusive exemplificatory purposes, examples are illustrated in relation to the use for tibial crest advancement in the event of rupture of the ACL and the use as a scaffold after ostectomy and for treatment of bone loss.

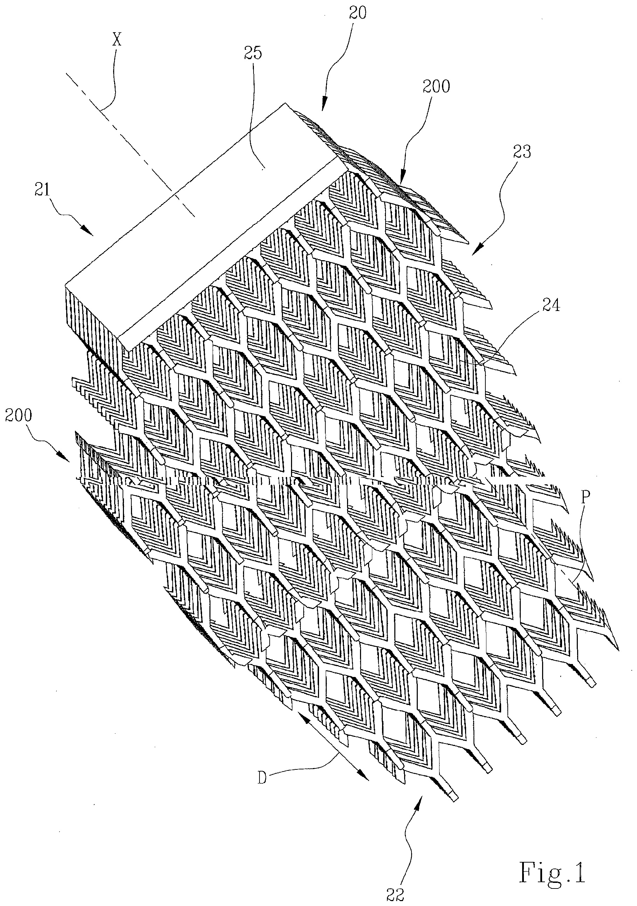

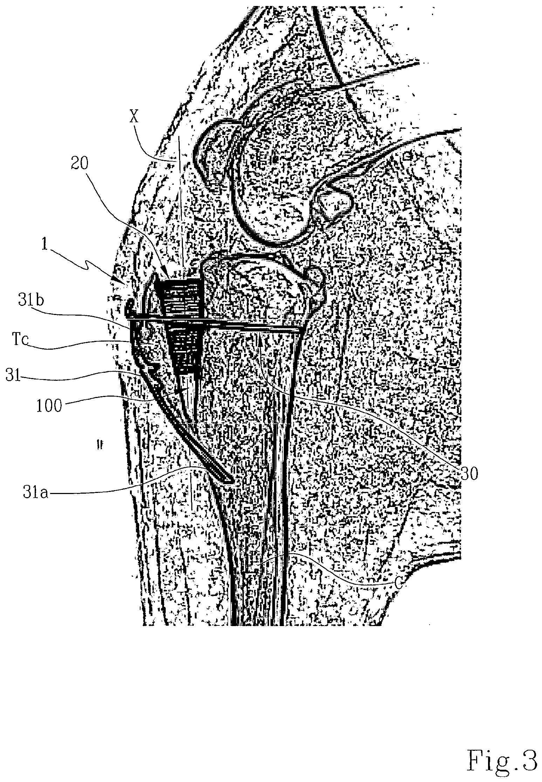

With reference to the appended figures, and in particular figure 3, 1 denotes in its entirety an implant for the advancement of a bone portion. In the example represented, the bone is represented by the tibia, and the advancement involves the tibial crest (TC). This does not alter the fact that the invention can be used for the advancement of a portion of another bone, both for veterinary and for human use.

A tibial crest advancement procedure for veterinary use entails osteotomising the tibial crest and advancing it to generate a longitudinal slit 100 in the tibia, as illustrated, for example, in figure 3.

The separating body 20 is made of biocompatible material, preferably titanium, and is suitable for insertion into an osteotomy on the front plane 100 of the tibia.

With reference to figure 3 corresponding to an engagement configuration of the implant 1, "X" indicates a longitudinal direction of the separating body 20, which is substantially parallel or slightly inclined relative to the direction of the major or longitudinal axis of the tibia.

During the tibial crest advancement procedure, the separating body 20 is introduced into the longitudinal osteotomy 100 along a direction transverse to the longitudinal direction "X".

Along the longitudinal direction "X", the separating body 20 extends between an upper portion 21 and a lower portion 22, which are such as to define a wedge-like conformation of the separating body itself. For example, the upper portion 21 has a greater extent than the lower portion 22 with reference to a direction transverse to the longitudinal direction "X". Therefore, during the tibial crest advancement intervention, the separating body 20 is introduced into the longitudinal osteotomy until being introduced completely inside the osteotomy itself.

The separating body 20 comprises a main section 23 having a plurality of openings 24 extending along a passage axis "P" disposed according to a direction transverse to the longitudinal direction "X", with reference to the engagement configuration of the implant. The passage axis "P" is, for example, perpendicular to the longitudinal direction "X".

The openings 24 are suitable for favouring osteo-integration. The openings 24 are disposed parallel to one another and form a mesh-like or sponge-like structure suitable for ensuring osteo-integration and hence the stabilisation of the implant itself. This structure is suitable for carrying cells, proteins or other biological material suitable for stimulating the bone response where the conditions may require it.

The three-dimensional structure can be easily cut to better adapt it to the specific conditions of individual procedures.

In a cross section perpendicular to the passage axis "P", the openings 24 have an elongate passageway, with its greater dimension "D" disposed along the longitudinal direction "X". Preferably, the openings have a hexagonal passageway.

In accordance with a possible embodiment, the openings 24 are disposed along rows and preferably the openings 24 of two adjacent rows alternate with each other.

Advantageously, the separating body has a reinforcement plate 25 disposed at the upper portion 21 thereof, transversely to the longitudinal direction "X". In accordance with a possible embodiment, the reinforcement plate 25 extends transversely to the longitudinal direction "X" on the upper portion 21 of the separating body 20. The reinforcement plate 25 can extend over a length that is smaller than the transverse extent of the upper portion 21, or else it can extend over the whole upper portion 21.

In particular, the reinforcement plate 25 has, transversely to the longitudinal direction "X", a rectangular shape. In a further possible embodiment, the reinforcement plate 25 can extend to an area of greater width than the upper portion 21. For example, the reinforcement plate 25 can also extend to lateral areas 200 of the separating body.

In a possible embodiment, the reinforcement plate 25 has one or more openings, structured so as to favour the oste-ointegration.

In accordance with a possible embodiment, the separating body 20 is produced by electron beam melting, both at the main section 23 and at the reinforcement plate 25.

In accordance with a possible embodiment, the implant 1 comprises a fastening body, configured to fasten the separating body 20 to the bone. In a possible embodiment, the fastening body comprises at least one fastening pin or nail 30.

The fastening pin or nail 30 is suitable for passing through one of the openings 24. In fact, during the tibial crest advancement surgery, it is envisaged to insert the pin or nail 30 according to an antero-posterior direction by passing through an opening 24 of the separating body 20. Thanks to the elongate passageway of the opening 24, it is possible to incline the pin or nail according to the anatomy of the tibia and/or the characteristics of the osteotomy, thereby preventing any positioning harmful to the joint structures and surrounding tissues. Furthermore, the fastening pin or nail 30 can be inserted into the tibial crest TC in the area of insertion of the patellar tendon, in which the bone is particularly solid and compact, so that the pin or nail 30 is positioned in a very stable manner.

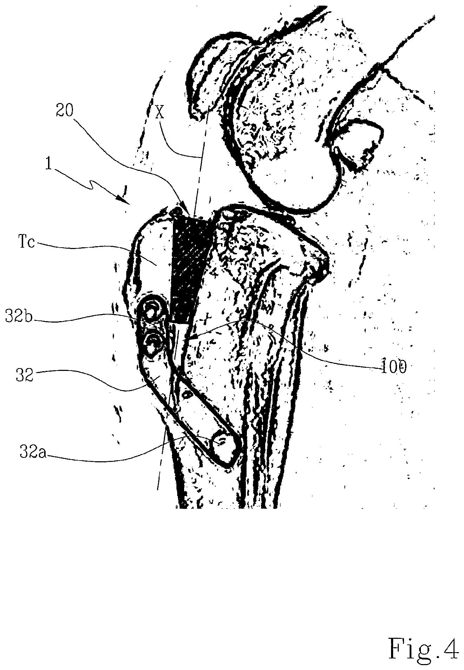

In a possible alternative embodiment, the fastening body comprises a cerclage 31 or a plate 32 fastenable to the tibial crest by means of screws, as shown respectively in figures 3 and 4.

At a distal end 31a, the cerclage 31, configured as a tension band, can be fastened to the bone distally to the osteotomy, thus permitting rotation on an anteroposterior plane. In the embodiment of figure 3, the cerclage 31 is fastened to the tibial crest TC by means of a nail 30, disposed through the proximal portion of the crest 31b and through one of the openings 24 of the separating body 20. As already highlighted, thanks to the elongate passageway of the opening 24, it is possible to incline the nail 30 in the most suitable way to adapt it to the anatomy of the tibia and/or the characteristics of the longitudinal slit, thereby preventing any positioning harmful to the joint structures and surrounding tissues, such as, for example, breakage of the tibial plateau. Furthermore, the presence and conformation of the openings 24 enable the cerclage 31 to be positioned with only the edge thereof placed at the distal end 31a. The cerclage 31 remains free to rotate in the distal bone anchorage hole, so that the separating body 20 can be compressed between the tibial crest TC and the tibia by the force generated by the tension of the patellar tendon, thus speeding up the process of osteointegration. At a distal end 32a, the plate 32 can be fastened to the bone by means of a screw that allows rotation on an antero-posterior plane. At the proximal end 32b, the plate can be fastened to the tibial crest TC by means of screws or another means. The plate 32 thus remains free to rotate around the screw located at the distal end 32a, so that the separating body 20 is compressed between the tibial crest TC and the tibia by the force generated by the tension of the patellar tendon, thus speeding up the process of osteo-integration.

In the case of figure 3, the insertion of a nail or pin 30 contributes to preventing migratory phenomena of the separating body 20. For this purpose it is also possible to use several nails or pins 30.

In this context, the architecture of figure 4 could also include one or more nails or pins 30 for the purpose of stabilising the separating body 20, thus averting migratory phenomena thereof.

Further fastening means, available in the sector, are also possible for fastening the separating body to the bone.

The implants 1 according to the present invention can comprise a plurality of separating bodies 20 and/or pins 30 or other fastening means having different shapes and size so as to be adapted to the anatomy of the subject to be operated on.

Thanks to the provision of a reinforcement plate 25, it is possible to avoid the risk of collapse of the separating body while maintaining the mesh-like or sponge-like structure, which ensures the correct, stable fastening of the graft to the bone structure.

Thanks to the provision of an elongate shape of the opening 24, it is possible, also in the case of tibial crest advancement interventions, to use a technique of fastening by means of a pin or nail, inserting the pin or nail according to the most correct angle.

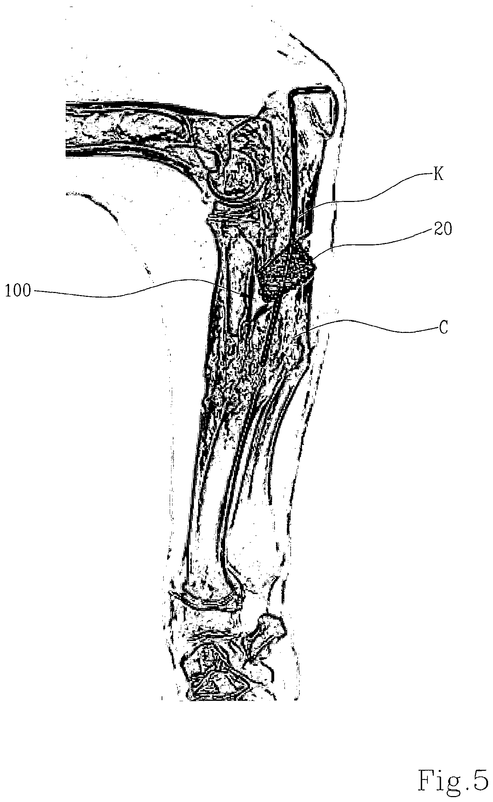

A further example of a possible use of the implant according to the present invention is illustrated in figure 5. In this example, the implant is used to support a bone deficit of the ulna. The implant is cut intraoperatively to enable the best adaptation thereof to the specific conditions, and then secured in place by passing a Kirschner wire of small size through the openings P. Thanks to its three-dimensional structure it is possible to insert a cancellous bone graft, which is kept in place and protected from excessively early loads by the structure of the implant.

With reference to the example of figure 5, all of the previously highlighted features are valid, but in the specific example the feature whereby the implant can be moulded to the specific characteristics of the site of use is particularly important. In the representation, the implant is used for the function of support and of carrying osteo-inductive cells, i.e. cancellous graft. In view of the particular characteristics of the engagement site, the implant has been cut to the size of the patient's bone to enable the correct conformation thereof.

## Claims

1. An implant (1) for maintaining bone diastasis after osteotomy of the tibial crest during a tibial crest advancement intervention, the intervention comprising the steps of osteotomy of the tibial crest and the subsequent cranial advancement thereof to generate a space between the tibial metaphysis and the tibial crest,
the implant comprising a separating body (20) made of a biocompatible material, the separating body (20) being suitable for insertion into said space and extending, with reference to an engagement configuration of said implant, along a longitudinal direction (X) between an upper portion (21) and a lower portion (22), said separating body (20) comprising:
a main section (23) having a plurality of openings (24) disposed parallel to one another, each of them extending along a passage axis (P) disposed in a direction transverse to said longitudinal direction (X), with reference to the engagement configuration of said implant, said openings (24) being suitable for promoting osteo-integration,
and
at least one reinforcement plate (25) disposed in the upper portion (21) and/or on lateral surfaces (200), transversely to said longitudinal direction (X);
**characterised in that** in a cross section perpendicular to said passage axis (P), said openings (24) have an elongate passageway with a greater dimension (D), which is disposed along said longitudinal direction (X).

2. The implant according to claim 1, wherein said reinforcement plate (25) has one or more openings.

3. The implant according to one or more of the preceding claims, wherein said reinforcement plate (25) extends transversely to said longitudinal direction (X) on the upper portion (21) and/or lateral portion (200) of the separating body (20).

4. The implant according to one or more of the preceding claims, wherein transversely to said longitudinal direction (X), said upper portion (21) has a greater extent than said lower portion (22), so that said separating body (20) has a wedge-like conformation.

5. The implant according to claim 1, wherein said openings (24) are disposed along rows, and wherein the openings of two adjacent rows alternate with each other.

6. The implant according to claim 1, wherein said openings (24) have a hexagonal passageway.

7. The implant according to one or more of the preceding claims, further comprising a fastening pin or nail (30) suitable for passing through said opening (24).

8. The implant according to claim 7, comprising a cerclage configured as a tension band (31), which has a distal end (31a) and a proximal end (31b) and is designed to be fastened to the bone portion (TC), at the proximal end (31b), by means of the pin or nail (30), and to be anchored to the bone (C) at the distal end (31a), with the possibility of rotation on an anteroposterior plane.

9. The implant according to claim 7, comprising a plate (32) which has a distal end (32a), fastenable to the bone (C), with the possibility of rotation on an anteroposterior plane, and a proximal end (32b), fastenable to the bone portion (TC).

10. The implant according to one or more of the preceding claims, wherein said separating body (20) is produced by electron beam melting.

11. The implant according to one or more of the preceding claims, wherein the three-dimensional structure is configured to carry cellular or protein material prepared so as to obtain an effect of stimulating bone formation.

12. The implant according to one or more of the preceding claims, wherein the three-dimensional structure can be dimensionally and morphologically adapted to the specific situation of the patient by cutting the three-dimension meshes.

13. The implant according to one or more of the preceding claims, comprising one or more pins or nails (30) configured to secure the positioning of the separating body (20), thus preventing migratory phenomena thereof.

## Patentansprüche

1. Implantat (1) zur Aufrechterhaltung von Knochendiastase nach Osteotomie der Scheinbeinvorderkante während eines Eingriffs zum Vorrücken der Scheinbeinvorderkante, wobei der Eingriff die Schritte der Osteotomie der Scheinbeinvorderkante und deren kranialen anschließenden Vorrückens umfasst, um einen Raum zwischen der Scheinbeinmetaphyse und der Scheinbeinvorderkante zu erzeugen,
wobei das Implantat einen Trennkörper (20) aus einem biokompatiblen Material umfasst, wobei der Trennkörper (20) zum Einsetzen in den Raum geeignet ist und sich unter Bezugnahme auf eine Eingriffskonfiguration des Implantats entlang einer Längsrichtung (X) zwischen einem oberen Abschnitt (21) und einem unteren Abschnitt (22) erstreckt,
wobei der Trennkörper (20) umfasst:
einen Hauptabschnitt (23) aufweisend eine Vielzahl an parallel zueinander angeordneten Öffnungen (24), von denen sich eine jede entlang einer Durchgangsachse (P) erstreckt, die in einer Richtung quer zur Längsrichtung (X) angeordnet ist, unter Bezugnahme auf die Eingriffskonfiguration des Implantats, wobei die Öffnungen (24) zur Förderung der Osteointegration geeignet sind, und
mindestens eine Verstärkungsplatte (25), die im oberen Abschnitt (21) und/oder auf Seitenflächen (200) quer zur Längsrichtung (X) angeordnet ist;
**dadurch gekennzeichnet, dass** in einem Querschnitt senkrecht zur Durchgangsachse (P) die Öffnungen (24) einen länglichen Durchgang mit einer größeren Abmessung (D) aufweisen, der entlang der Längsrichtung (X) angeordnet ist.

2. Implantat nach Anspruch 1, wobei die Verstärkungsplatte (25) eine oder mehrere Öffnungen aufweist.

3. Implantat nach einem oder mehreren der vorhergehenden Ansprüche, wobei sich die Verstärkungsplatte (25) quer zur Längsrichtung (X) auf dem oberen Abschnitt (21) und/oder seitlichen Abschnitt (200) des Trennkörpers (20) erstreckt.

4. Implantat nach einem oder mehreren der vorhergehenden Ansprüche, wobei der obere Abschnitt (21) quer zur Längsrichtung (X) eine größere Ausdehnung aufweist als der untere Abschnitt (22), so dass der Trennkörper (20) eine keilartige Konformation aufweist.

5. Implantat nach Anspruch 1, wobei die Öffnungen (24) entlang Reihen angeordnet sind und wobei die Öffnungen zweier benachbarter Reihen miteinander abwechseln.

6. Implantat nach Anspruch 1, wobei die Öffnungen (24) einen sechseckigen Durchgang aufweisen.

7. Implantat nach einem oder mehreren der vorhergehenden Ansprüche, ferner umfassend einen Befestigungsstift oder Nagel (30), der geeignet ist, um durch die Öffnung (24) zu verlaufen.

8. Implantat nach Anspruch 7, umfassend eine als Spannband (31) konfigurierte Cerclage, die ein distales Ende (31a) und ein proximales Ende (31b) aufweist und die ausgelegt ist, um mit dem Knochenabschnitt (TC) am proximalen Ende (31b) mittels des Stifts oder Nagels (30) befestigt zu werden und um am distalen Ende (31a) mit dem Knochen (C) verankert zu werden, mit der Möglichkeit einer Drehung auf einer antero-posterioren Ebene.

9. Implantat nach Anspruch 7, umfassend eine Platte (32), die ein distales Ende (32a), das mit dem Knochen (C) befestigt werden kann, mit der Möglichkeit der Drehung auf einer antero-posterioren Ebene, und ein proximales Ende (32b), das mit dem Knochenabschnitt (TC) befestigt werden kann, aufweist.

10. Implantat nach einem oder mehreren der vorhergehenden Ansprüche, wobei der Trennkörper (20) durch Elektronenstrahlschmelzen hergestellt wird.

11. Implantat nach einem oder mehreren der vorhergehenden Ansprüche, wobei die dreidimensionale Struktur konfiguriert ist, um Zell- oder Proteinmaterial zu tragen, das vorbereitet wurde, um einen Effekt zur Stimulierung der Knochenbildung zu erhalten.

12. Implantat nach einem oder mehreren der vorhergehenden Ansprüche, wobei die dreidimensionale Struktur durch Schneiden der dreidimensionalen Maschen dimensional und morphologisch an die spezifische Situation des Patienten angepasst werden kann.

13. Implantat nach einem oder mehreren der vorhergehenden Ansprüche, umfassend einen oder mehrere Stifte oder Nägel (30), die konfiguriert sind, um die Positionierung des Trennkörpers (20) sicherzustellen, wodurch dessen Migrationsphänomene verhindert werden.

## Revendications

1. Implant (1) pour maintenir une diastase osseuse après une ostéotomie de la crête tibiale pendant une intervention d'avancement de la crête tibiale, l'intervention comprenant les étapes d'ostéotomie de la crête tibiale et l'avancement crânien ultérieur de celle-ci pour générer un espace entre la métaphyse tibiale et la crête tibiale,
l'implant comprenant un corps de séparation (20) composé d'un matériau biocompatible, le corps de séparation (20) étant adapté pour être introduit dans ledit espace et se prolongeant, en référence à une configuration d'engagement dudit implant, le long d'une direction longitudinale (X) entre une partie supérieure (21) et une partie inférieure (22),
ledit corps de séparation (20) comprenant :
une section principale (23) comportant une pluralité d'ouvertures (24) disposées parallèlement les unes aux autres, chacune d'elles se prolongeant le long d'un axe de passage (P) disposé dans une direction transversale à ladite direction longitudinale (X), en référence à la configuration d'engagement dudit implant, lesdites ouvertures (24) étant adaptées pour favoriser l'ostéo-intégration, et
au moins une plaque de renforcement (25) disposée dans la partie supérieure (21) et/ou sur des surfaces latérales (200), transversalement à ladite direction longitudinale (X) ;
**caractérisé en ce que** dans une section transversale perpendiculaire audit axe de passage (P), lesdites ouvertures (24) comportent un passage allongé, ayant une plus grande dimension (D), étant disposé le long de ladite direction longitudinale (X).

2. Implant selon la revendication 1, dans lequel ladite plaque de renforcement (25) comporte une ou plusieurs ouvertures.

3. Implant selon une ou plusieurs des revendications précédentes, dans lequel ladite plaque de renforcement (25) se prolonge transversalement à ladite direction longitudinale (X) sur la partie supérieure (21) et/ou la partie latérale (200) du corps de séparation (20).

4. Implant selon une ou plusieurs des revendications précédentes, dans lequel, transversalement à ladite direction longitudinale (X), ladite partie supérieure (21) possède une plus grande extension que ladite partie inférieure (22), de sorte que ledit corps de séparation (20) possède une conformation en forme de coin.

5. Implant selon la revendication 1, dans lequel lesdites ouvertures (24) sont disposées le long de rangées, et dans lequel les ouvertures de deux rangées adjacentes s'alternent les unes avec les autres.

6. Implant selon la revendication 1, dans lequel lesdites ouvertures (24) comportent un passage hexagonal.

7. Implant selon une ou plusieurs des revendications précédentes, comprenant de plus une cheville ou un clou (30) de fixation adapté pour passer à travers ladite ouverture (24).

8. Implant selon la revendication 7, comprenant un cerclage configuré, comme une bande de tension (31), comportant une extrémité distale (31a) et une extrémité proximale (31b) et étant conçu pour être fixé à la partie osseuse (TC), en correspondance de l'extrémité proximale (31b), au moyen de la cheville ou du clou (30), et pour être ancré à l'os (C) en correspondance de l'extrémité distale (31a), avec la possibilité de rotation sur un plan antéropostérieur.

9. Implant selon la revendication 7, comprenant une plaque (32) comportant une extrémité distale (32a) pouvant être fixée à l'os (C), avec la possibilité de rotation sur un plan antéropostérieur, et une extrémité proximale (32b), pouvant être fixée à la partie osseuse (TC).

10. Implant selon une ou plusieurs des revendications précédentes, dans lequel ledit corps de séparation (20) est produit par fusion par faisceau d'électrons.

11. Implant selon une ou plusieurs des revendications précédentes, dans lequel la structure tridimensionnelle est configurée pour porter un matériau cellulaire ou protéique préparé de manière à obtenir un effet de stimulation de la formation osseuse.

12. Implant selon une ou plusieurs des revendications précédentes, dans lequel la structure tridimensionnelle peut être adaptée dimensionnellement et morphologiquement à la situation spécifique du patient en coupant les mailles tridimensionnelles.

13. Implant selon une ou plusieurs des revendications précédentes, comprenant une ou plusieurs chevilles ou clous (30) configuré(e)s pour assurer le positionnement du corps de séparation (20), empêchant ainsi les phénomènes migratoires de celui-ci.
